# EUROPÄISCHE PATENTANMELDUNG

(11) **EP 1 357 114 A1**
(43) Veröffentlichungstag der Anmeldung: **29.10.2003**
(21) Anmeldenummer: 02022635.3
(22) Anmeldetag: 09.10.2002
(51) Int. Cl.: C07D 239/96, C07D 495/04, A61K 31/517, A61K 31/519, A61P 37/00

(54) **Substituierte Pyrimidin-2,4(1H,3H)-dione als Matrix Metalloproteinase (MMP) Inhibitoren**

(30) Priorität: 22.04.2002 DE 10217813
(71) Anmelder: IBFB GmbH Privates Institut für biomedizinische Forschung und Beratung, 04229 Leipzig (DE)
(72) Erfinder: Heinicke, Jochen, 04229 Leipzig (DE); Klausmeier, Uwe, 33330 Gütersloh (DE)
(74) Vertreter: Schüssler, Andrea, Dr.

(57) **Zusammenfassung**

Die Erfindung betrifft substituierte kondensierte Pyrimidin-2,4(1H,3H)-dione der allgemeinen Formel I, 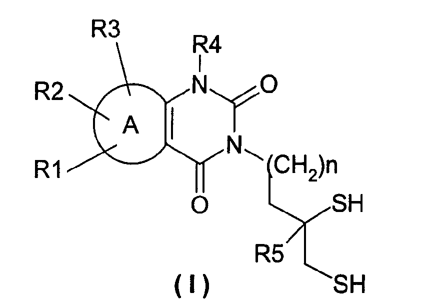 deren Tautomere, deren Stereoisomere, Gemische, Prodrugs und Salze, wobei in der Formel I die Indices bedeuten:
n = die Zahlen 0, 1 oder 2,
A = ein anellierter Benzoring oder ein anellierter fünf- bis siebengliedriger Cycloalkylring oder ein anellierter fünf- bis siebengliedriger Cycloheteroalkylring mit einem Sauerstoff- oder Schwefelatom oder ein über zwei Kohlenstoffatome gebundener Heteroaromat mit einem, zwei oder drei Stickstoff-, Sauerstoff- und/oder Schwefelatomen als Heteroatomen,
R1, R2, R3 = unabhängig voneinander Wasserstoff, Halogen, C1-C6 Alkyl, C1-C3-Alkylthio-, C6-C12 Aryl-, Nitro-, Carbamoyl-, C1-C4 Alkoxy, -CN, CF₃, NH₂ -, Carboxy-, C1-C4 Alkoxycarbonyl, C1-C4 N-alkyl-carbamoyl oder C1-C5 Alkenyl
R4 = Wasserstoff, C1-C8 Alkyl, C2-C5 Alkenyl, C2-C8 Alkinyl, C3-C10 Cycloalkyl-, C3-C10 Cycloalkenyl-, C3-C10 Cycloalkinyl-, [-(CH₂)₁₋₅-Z-(C1-C10 Alkyl) mit Z= O, S, C=O, S=O, SO₂], C6-C12 Aryl, C7-C12 Aralkyl,
R5 = Wasserstoff, Methyl, wobei
die für R1 bis R4 definierten Alkyl-, Alkylthio-, Alkoxy-, Alkoxycarbonyl-, N-alkylcarbamoyl-, Alkenyl-, Alkinyl-, Cycloalkyl-, Cycloalkenyl- und Cycloalkinylgruppen sowie die Z-Gruppe mono oder disubstituiert sein können durch Reste aus der Gruppe:

Carbamoyl-, Hydroxy, C1-C4 Alkoxy, -CN, SO₃H, CF₃, NH₂ , Carboxy-, C1-C4 Alkoxycarbonyl, C1-C4 N-alkyl-carbamoyl, C1-C4 N-dialkyl-carbamoyl, Phenyl oder Piperazinylcarboxy, wobei vorstehend genannten Alkylgruppen substituiert sein können durch ein oder zwei Hydroxygruppen, SO₃H, NH₂, C1-C4 alkylamino, C1-C4 dialkylamino, oder einen Heterocyclus.

Diese Verbindungen stellen Matrix Metalloproteinase-Inhibitoren dar und eignen sich daher zur Herstellung von Arzneimitteln gegen Krebs, Rheuma, Entzündungen und allergische Reaktionen.

## Beschreibung

Die Erfindung betrifft substituierte kondensierte Pyrimidin-2,4(1H,3H)-dione der allgemeinen Formel I, 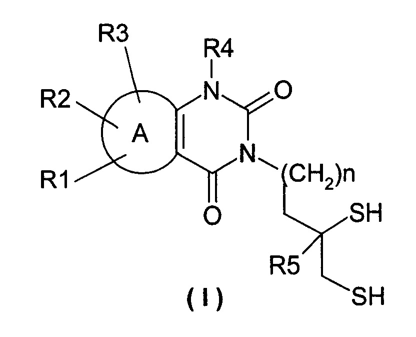 deren Tautomere, deren Stereoisomere, Gemische, Prodrugs und Salze, wobei in der Formel I die Indices bedeuten:
n = die Zahlen 0,1 oder 2,
A = ein anellierter Benzolring oder ein anellierter fünf- bis siebengliedriger Cycloalkylring oder ein anellierter fünf- bis siebengliedriger Cycloheteroalkylring mit mindestens einem Sauerstoff- oder Schwefelatom oder ein über zwei Kohlenstoffatome gebundener Heteroaromat mit einem, zwei oder drei Stickstoff-, Sauerstoffund/oder Schwefelatomen als Heteroatomen,
R1, R2, R3 = unabhängig voneinander Wasserstoff, Halogen, C1-C6 Alkyl, C1-C3-Alkylthio-, C6-C12-Aryl-, Nitro-, Carbamoyl-, C1-C4 Alkoxy, -CN, CF₃, NH₂ -, Carboxy-, C1-C4 Alkoxycarbonyl, C1-C4 N-alkyl-carbamoyl oder C1-C5 Alkenyl
R4 = Wasserstoff, C1-C8 Alkyl, C2-C5 Alkenyl, C2-C8 Alkinyl, C3-C10 Cycloalkyl-, C3-C10 Cycloalkenyl-, C3-C10 Cycloalkinyl-, [-(CH₂)₁₋₅-Z-(C1-C10 Alkyl) mit Z= O, S, C=O, S=O, SO₂], C6-C12 Aryl, C7-C12 Aralkyl,
R5 = Wasserstoff, Methyl

Die für R1 bis R4 definierten Alkyl-, Alkylthio-, Alkoxy-, Alkoxycarbonyl-, N-alkyl-carbamoyl-, Alkenyl-, Alkinyl-, Cycloalkyl-, Cycloalkenyl- und Cycloalkinylgruppen sowie die Z-Gruppe können vorzugsweise mono- oder disubstituiert sein durch Reste aus der Gruppe:
Carbamoyl-, Hydroxy, C1-C4 Alkoxy, -CN, SO₃H, CF₃, NH₂ , Carboxy-, C1-C4 Alkoxycarbonyl, C1-C4 N-alkyl-carbamoyl, C1-C4 N-dialkyl-carbamoyl, Phenyl oder Piperazinylcarboxy oder einen Heterocyclus wobei vorstehend genannten Alkylgruppen substituiert sein können durch ein oder zwei Hydroxygruppen, SO₃H , NH₂, C1-C4 alkylamino, C1-C4 dialkylamino, oder einen Heterocyclus.

Auf dem Gebiet der Entwicklung effizienter, niedermolekularer und nichtproteinogener MMP (Matrix Metalloproteinase) Inhibitoren wird weltweit intensiv geforscht. Es ist bekannt, dass physiologisch die enzymatische Aktivität der MMPs unter einer strikten, abgestimmten Regulation zwischen Aktivierung und Hemmung steht. Hierzu existieren im Örganismus spezielle Proteine, die sog. Tissue Inhibitors of Matrixmetalloproteinases (TIMP's), die in der Lage sind, die Aktivität von MMPs schnell und effizient zu hemmen (*Nagase, H. et al.: Engineering of selective TIMPs. 1-11; In: Inhibition of Matrix Metalloproteinases - Therapeutic Application (Eds. Greenwal,d RA., Zucker, S., Golub, LM.) Ann NY Acad Sci* 878 (1999). Eine ungebremste enzymatische Aktivität dieser Enzyme führt besonders bei den rheumatischen Erkrankungen zum Abbau der Knorpelsubstanz und - pathologisch bedeutsam - zu chronisch-schmerzhaften Veränderungen an den Gelenken *(Goldbach-Mansky, R. et al.: Active synovial matrix metalloproteinase-2 is associated with radiographic erosions in patients with early synovitis. Arthritis Res 2 (2000) 145-153).*

Ein weiteres Beispiel für die pathologische Wirkung von MMPs ist bei der Invasion und Metastasierung von Tumoren gegeben. Freigesetzte und aktivierte MMPs schneiden einen Weg durch das dichtes kollagenes Bindegewebe und insbesondere auch durch die Basalmembran der Gefäße und ermöglichen es dadurch den Krebszellen aus dem Tumorverband auszuwandern, in das Gefäßsystem einzuwandern und an anderer Stelle Tochtergeschwülste zu bilden. MMPs spielen eine weitere entscheidende Rolle bei der Blutgefäßversorgung des wachsenden Tumors, indem sie den Weg für die neugebildeten Blutgefäße durch das kollagene Bindegewebe freischneiden und somit für diese Vaskularisation des wachsenden Tumors verantwortlich sind *(Shapiro, SD.: Matrix metalloproteinase degradation of extracellular matrix: biological consequences. Current Opinion in Cell Biology 10 (1998) 602-608).*

Als weiteres relevantes medizinisches Ergebnis unzureichend gebremster Wirkung von MMPs ist das UV-induzierte Erythem zu nennen, das u.a. als Folge einer intensiven Sonnenbestrahlung auftritt. Durch die energiereichen UV-Strahlen des Sonnenlichtes bzw. von Bräunungsgeräten werden u.a. die inaktiven Prokollagenasen in der bestrahlten Haut aktiviert, die in der Folge Kollagen des Bindegewebes und der Blutkapillaren spalten und dadurch für die Symptome eines Sonnenbrandes verantwortlich zeichnen.

Bei diesen exemplarisch aufgezeigten pathologischen Wirkungen der ungebremsten enzymatischen Aktion von MMPs, können deren Folgeerscheinungen durch stabile MMP- Inhibitoren verhindert bzw. wesentlich gemildert werden. Es ist faszinierend, die Vorstellung zu realisieren, diese Enzyme durch spezifische Inhibitoren gezielt zu hemmen, um dadurch zum Beispiel eine fortschreitende Knorpeldestruktion bei Erkrankungen des rheumatischen Formenkreises zu unterbrechen, oder das Wachstum bzw. die Metastasierung von Tumoren zu verhindern.

Es sind bereits zahlreiche Verfahren zur Gewinnung von Verbindungen mit MMP-inhibierender Wirkung bekannt. Die Wirkstoffe der ersten Generation besitzen in aller Regel eine proteinogene Struktur und weisen eine strukturelle Verwandtschaft zu natürlichen Inhibitoren auf, bei denen es sich um spezielle Proteine handelt. Diese proteinogenen oder pseudoproteinogenen Substratanaloga besitzen als Strukturelement eine zinkbindende Gruppe, die das Zinkion im aktiven Zentrum der MMPs chelatisiert.

Bezüglich einer therapeutischen Anwendung weisen alle derartigen proteinogenen bzw. pseudoproteinogenen Wirkstoffe eine Reihe von Nachteilen auf, wie ungenügende Resorbierbarkeit, in aller Regel kurze Halbwertszeiten, eine nur geringe Stabilität, sowie häufig unerwünschte Nebenwirkungen *(Inhibition of Matrix Metalloproteinases - Therapeutic Application (Eds. Greenwald, RA., Zucker, S., Golub, LM.) Ann NY Acad Sci* 878 *(1999)).*

Weitere Entwicklungen auf diesem Gebiet erbrachten beispielsweise Phosphonamid-Inhibitoren, Piperazin-Inhibitoren, Sulfonamid-Inhibitoren, Carbamat-Inhibitoren, Diazepin-Inhibitoren, Tetracyclin-Inhibitoren und nicht zuletzt Hydroxamat-Inhibitoren *(Skotnick,i JS. et al.: Design and synthetic considerations of matrix metalloproteinase inhibitors. 61-72 In: Inhibition of Matrix Metalloproteinases - Therapeutic Application (Eds. Greenwald, RA., Zucker, S., Golub, LM.) Ann NY Acad Sci 878* (1999)). Die meisten dieser entwickelten Inhibitoren weisen zwar *in vitro* beeindruckende Hemmwirkungen und Spezifitäten auf, zeigten aber bei *in vivo* Anwendungen im Tierversuch und beim Menschen eine Reihe von schwerwiegenden Nachteilen. Im Vordergrund standen hierbei zytotoxische Reaktionen auf eine Vielzahl von Zellen, eine schlechte Bioverfügbarkeit und unerwünschte Nebenwirkungen, insbesondere eine negative Beeinflussung des Bewegungsapparates.

Es sind auch unterschiedliche Substanzklassen untersucht worden auf die Wirkung ihrer Vertreter als MMP-Inhibitoren. So wurde bereits vorgeschlagen, Thiazolo[2,3-b]chinazolinone, Oxazolo[2,3-b]chinazolinone, Imidazo[2,1-b]chinazolinone, Benzothiadiazin-5,5-dioxide (DE 100 46 728), 1-Dimercaptoalkylchinazolin-2,4(1H,3H)-dione, mehrcyclische Pyrimidin-2,4(1H,3H)-dione mit funktionalisierten Alkylresten in 1- und/oder 3-Position (WO 01/14344) als MMP-Inhibitoren einzusetzen.

Trotzdem besteht weiterhin ein Bedarf an Arzneimitteln mit nichtproteinogener Struktur, welche die Nachteile der Wirkstoffe, die bisher am Markt sind, nicht aufweisen. Insbesondere besteht Bedarf an neuen Wirkstoffen mit MMP-inhibierender Wirkung, die ausreichend stabil und gut resorbierbar sind, bessere pharmakokinetische Eigenschaften besitzen und vor allen Dingen keine unerwünschten Nebenwirkungen und zytotoxische Reaktionen aufweisen.

Es besteht deshalb die Aufgabe, neue chemische Substanzen nichtproteinogener Struktur aufzufinden, die als Matrix Metalloproteinase Inhibitoren geeignet sind. Sie sollen sich von den bisher bekannten Inhibitoren durch günstige inhibitorische Wirkung bei geringen Nebenwirkungen vorteilhaft unterscheiden.

Es ist weiterhin das Ziel der Erfindung, Arzneimittel auf der Basis von MMP Inhibitoren zur Behandlung zahlreicher Erkrankungen, wie z.B. Krebs, Rheuma, unspezifische Entzündungsreaktionen u.a. beim Sonnenbrand und allergischen Reaktionen zu entwickeln.

Die Aufgabe wird dadurch gelöst, daß Pyrimidindione der allgemeinen Formel I hergestellt werden und es gefunden wurde, daß die neuen Verbindungen die gewünschte Wirkung als MMP-Inhibitoren aufweisen.

Die Aufgabe wird insbesondere durch substituierte kondensierte Pyrimidin-2,4(1 H,3H)-dione der allgemeinen Formel I, 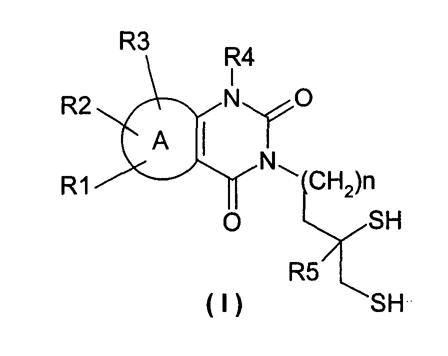 deren Tautomere, deren Stereoisomere, Gemische, Prodrugs und Salze gelöst, wobei in der Formel I die Indices bedeuten:
n = die Zahlen 0, 1 oder 2,
A = ein anellierter Benzoring oder ein anellierter fünf- bis siebengliedriger Cycloalkylring oder ein anellierter fünf- bis siebengliedriger Cycloheteroalkylring mit einem Sauerstoff- oder Schwefelatom oder ein über zwei Kohlenstoffatome gebundener Heteroaromat mit einem, zwei oder drei Stickstoff-, Sauerstoff- und/oder Schwefelatomen als Heteroatomen,
R1, R2, R3 = unabhängig voneinander Wasserstoff, Halogen, C1-C6 Alkyl, C1-C3-Alkylthio-, C6-C12-Aryl-, Nitro-, Carbamoyl-, C1-C4 Alkoxy, -CN, CF₃, NH₂ -, Carboxy-, C1-C4 Alkoxycarbonyl, C1-C4 N-alkyl-carbamoyl oder C1-C5 Alkenyl
R4 = Wasserstoff, C1-C8 Alkyl, C2-C5 Alkenyl, C2-C8 Alkinyl, C3-C10 Cycloalkyl-, C3-C10 Cycloalkenyl-, C3-C10 Cycloalkinyl-, [-(CH₂)₁₋₅-Z-(C1-C10 Alkyl) mit Z= O, S, C=O, S=O, SO₂], C6-C12 Aryl, C7-C12 Aralkyl,
R5 = Wasserstoff, Methyl

Die als Rest R1, R2, R3 oder R4 genannten Alkyl-, Alkoxy-, Alkylthio-, Alkoxycarbonyl-, N-alkyl-carbamoyl-, Alkenyl-, Alkylcarboxy- oder Alkylsulfonyl-Gruppen können verzeigt oder unverzeigt bzw. offenkettig oder cyclisch sein. Desweiteren können sie vorzugsweise Substituenten, wie z.B. Halogenatome, Cyano-, Carboxy-, Carbonyl-, Nitril-, Carbamoyl-, C1-C4-Alkoxy, Phenyl, C1-C4 Alkoxycarbonyl-, C1-C4 Alkylcarboxy-, C1-C4 N-Alkyl-carbamoyl-, C1-C4 N-Dialkylcarbamoyl-, Hydroxy-, Nitro.-, SO₃H-, Ether-, Sulfamoyl-, C1-C4 N-Alkylsulfamoyl, C1-C4 Dialkylsulfamoyl, -CO-R (mit: R = OH, O-Alkyl, NH-Alkyl), Trifluormethyl-Gruppierungen oder weitere offenkettige oder cyclische Gruppierungen mit Heteroatomen (z.B. mit N, S und/oder O) aufweisen, bei denen ggf. die Heteroatome Teil eines Heterocyclus sein können. Das mögliche Vorhandensein von vorgenannten Substituenten gilt für alle als Reste R1, R2, R3 oder R4 genannten Gruppen (soweit substituierbar), insbesondere für die dort genannten Alkyl-, Cycloalkyl-, Alkenyl-, Cycloalkyl- und C6-C12 ArylGruppen sowie die "Z"-Gruppe enthaltende Gruppierung.

Unter "Halogen" wird Fluor, Chlor, Brom oder lod verstanden.

Als Rest "A" sind ein annelierter Benzol- oder Thiophenring bevorzugt. Desweiteren sind bevorzugt Pyrrol-, Thiazol-, Furan-, Imidazol-, Pyrazol-, Pyridin-, Pyrimidinoder Pyrazin-Ringe/Ringsysteme.

Die Begriffe "anneliert" und "ankondensiert" werden synonym verwendet.

Unter C1-C4 Alkyl sind Methyl, Ethyl., n-Propyl-, Isopropyl-, n-Butyl, Isobutyl., sek. Butyl. und tert-Butyl zu verstehen.

Unter C1-C8 Alkyl sind u.a. Methyl, Ethyl., n-Propyl-, Isopropyl-, n-Butyl, Isobutyl., sek. Butyl., tert-Butyl, n-Pentyl, Isopentyl-, Neopentyl, n-Hexyl, 2-Methylpentyl, 3-Methylpentyl, 2,2-Dimethylbutyl, 2,3-Dimethylbutyl, n-Heptyl, 2-Methylhexyl, 3-Methylhexyl, 2,2-Dimethylpentyl, 2,3-Dimethylpentyl, 2,4-Dimethylpentyl-, 3,3-Dimethylpentyl, 3-Äthylpentyl, 2,2,3- Trimethylbutyl-, n-Octyl-, 2-Methylhepyl, 3-Methylhepyl, 4-Methylhepyl-, 2,2-Dimethylhexyl-, 2,3-Dimethylhexyl-, 2,4-Dimethylhexyl-, 3,3-Dimethylhexyl-, 3,4-Dimethylhexyl, 4,4-Dimethylhexyl-, 2-Äthylhexyl, 3-Äthylhexyl-, 4-Äthylhexyl-, 2,2,3-Trimethylpentyl-, 2,3,3-Trimethylpentyl, usw. sowie deren cyclische Äquivalente zu verstehen.

Unter C2-C5 Alkenyl sind u.a. Ethen, Propen, 1-Buten, (cis/trans)-2-Buten, 2-Methylpropen, 1-Penten, 2-Penten, 2-Methyl-1-buten, 3-Methyl-1-buten und 2-Methyl-2-buten sowie deren cyclische Äquivalente zu verstehen.

Unter C2-C8 Alkinyl sind u.a. Acetylen, Propin, 1-Butin, 2- Butin, 1-Pentin, 2-Pentin, 3-Methyl-1-butin-, 1-Hexin, 2-Hexin, 3-Hexin, 3,3-Dimethyl-1-butin, 1-Heptin, 2-Heptin, 3-Heptin, 1-Octin, 2-Octin, 3-Octin und 4-Octin sowie deren cyclische Äquivalente zu verstehen.

Unter C1-C4 Alkoxy sind Methoxy, Ethoxy, Propoxy, Isopropoxy, Butoxy, Isobutoxy, sek-Butoxy- und tert-Butoxy zu verstehen.

Unter C6-C12 Aryl sind Phenyl-, Tolyl-, Xylyl-, Naphthyl-, Biphenyl-Ringsysteme zu verstehen.

Innerhalb der obigen Formel (I) gibt es folgende bevorzugte Verbindungen:

(a) dimercaptoalkylsubstituierte kondensierte Pyrimidin-2,4(1H,3H)-dione der allgemeinen Formel (la), 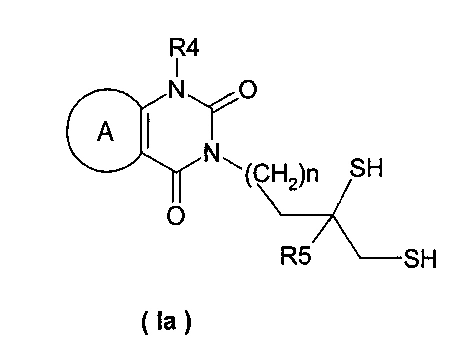 wobei in der Formel (la) die Indices bedeuten:
- n: = die Zahlen 0 oder 1
- R4: = Wasserstoff, C1-C8 Alkyl, C2-C5 Alkenyl, C2-C5 Alkinyl, [-(CH₂)₁₋₃-Z-(C1-C5 Alkyl) mit Z= O, S, C=O, S=O, SO₂], C7-C12 Aralkyl, wobei die Alkyl-, Alkenyl-, Alkinylgruppen sowie die Z-Gruppe mono oder disubstituiert sein können durch Reste aus der Gruppe: Carbamoyl-, Hydroxy, C1-C2 Alkoxy, -CN, SO₃H, CF₃, Carboxy-, C1-C4 Alkoxycarbonyl, C1-C4 N-alkyl-carbamoyl, C1- C4 N-dialkyl-carbamoyl oder Piperazinylcarboxy und vorstehend genannte Alkylgruppen substituiert sein können durch ein oder zwei Hydroxygruppen, SO₃H, NH₂, C1-C4 Alkylamino, C1-C4 Dialkylamino, oder einen Heterocyclus.
- R5: = ein Wasserstoffatom oder eine Methylgruppe
- A: = ein anellierter Benzoring unsubstituiert oder monosubstituiert, disubstituiert oder trisubstituiert mit Resten, die gleich oder verschiedenartig sein können aus der Gruppe: Halogen, C1 bis C3- Alkyl, C1 bis C3 Alkoxy, Trifluormethyl, Nitro-, Carbamoyl-, -NH₂ , Carboxy-, C1-C4 Alkoxycarbonyl, C1-C4 N-alkylcarbamoyl, Arylalkylcarbamoyl, Arylalkyloxycarbonyl, Het-alkylcarbamoyl und Het-alkyloxycarbonyl mit Aryl= Phenyl, Tolyl, Pyridyl, Pyrazinyl, Pyrimidinyl, Pyrrolyl, Pyrazolyl, Imidazolyl sowie Het= Morpholinyl, Piperazinyl, Piperidinyl, Pyrrolidinyl, Imidazolidinyl
oder ein anellierter fünf- bis siebengliedriger Cycloalkylring
oder ein anellierter fünf- bis siebengliedriger Cycloheteroalkylring mit einem Sauerstoff- oder Schwefelatom
oder ein über zwei Kohlenstoffatome gebundener Heteroaromat, dieser sechsgliedrig mit einem, zwei oder drei Stickstoffatomen als Heteroatomen oder
fünfgliedrig mit einem Stickstoffatom oder
fünfgliedrig mit einem Sauerstoff - oder Schwefelatom oder
fünfgliedrig mit einem Stickstoffatom sowie einem Sauerstoff- oder einem Schwefelatom oder
fünfgliedrig mit zwei Stickstoffatomen als Heteroatomen oder fünfgliedrig mit zwei Stickstoffatomen und einem Schwefelatom oder fünfgliedrig mit drei Stickstoffatomen als Heteroatomen,
wobei die vorstehend für A definierten Heteroaromaten durch Reste R6 mono-, di- oder trisubstituiert und die Substituenten gleich oder verschieden sein können
R6 = Halogen, C1 bis C6- Alkyl, C1 bis C3-Alkylthio, Cyano, Trifluormethyl, Benzyl, Phenyl, wobei die beiden letztgenannten aromatischen Reste wie folgt substituiert sein können:
C1 bis C3-Alkyl, Halogen, OR7, COR7, S02R7, OCOR8, COOR8, CONR8R9, SO2NR8R9, NR8R9 mit
R7 = H, C1 bis C4-Alkyl, C3 bis C6-Cycloalkyl, Phenyl,
R8/R9 = unabhängig voneinander H, Benzyl, letzteres unsubstituiert oder durch Methoxy mono-, di- oder trisubstituiert, C1 bis C4-Alkyl oder R8/R9 = als geradkettige Alkylgruppe gemeinsam einen Ring bildend mit einem Stickstoffatom einen fünfgliedrigen Ring, der ggf. ein weiteres Heteroatom N, O, S enthalten kann, oder gemeinsam einen sechsgliedrigen Heterocyclus bildend mit einem Stickstoffatom sowie ggf. einem weiteren Stickstoffatom oder einem Sauerstoffatom oder einem Schwefelatom, wobei Fünf- und/oder Sechsring im Kohlenstoffgerüst mit C1 bis C4 Alkyl oder mit Benzyl substituiert sein können;
R6 = für den Fall, daß A Thieno ist, außerdem ein anellierter C4- bis C7- Cycloalkylring, der ein Sauerstoff, ein Schwefel- oder ein Stickstoffatom enthalten kann, wobei das Stickstoffatom unsubstituiert oder substituiert ist mit C1 bis C4 Alkyl- , Phenyl oder Benzyl und die beiden letztgenannten Aromaten mit C1 bis C3 Alkyl, C1 bis C3 Alkoxy oder Halogen substituiert sein können, ferner kann für den Fall A= Thieno R6 außerdem sein: Carboxy, eine mit einem Heterocyclus substituierte Alkylgruppe C1-C5, ein Heterocyclus sowie eine C1-C5 N-Alkylcarbamoylgruppe, die mit Amino, Sulfo, Hydroxy, Dihydroxy oder einem Heterocyclus substituiert sein kann.

Die vorstehenden Erläuterungen hinsichtlich der chemischen Gruppierungen und möglichen Substitutionen gelten für die Verbindungen der Formel I(a) entsprechend.

Unter die Erfindung fallen auch Verbindungen der allgemeinen Formeln I und I(a) in Form ihrer Stereoisomere, Racemate, Enantiomere, Diastereomere, oder ihrer physiologisch verträglichen Salze mit anorganischen oder organischen Säuren bzw. Basen, sowie Gemische dieser Verbindungen. Gegenstand vorliegender Erfindung sind ferner Prodrugs der Verbindungen der allgemeinen Formeln I, I(a) oder I(b) in denen die Mercaptofunktion nach dem Fachmann an sich bekannten Verfahren (vgl. hierzu*: Higuchi, T., Stella, V., Prodrugs as Novel Delivery Systems, Vol. 14 of the A. C. S. Symposium Series; Roche.E.B., ed., Bioreversible Carriers in Drug Design, American Pharmaceutical Association and Pergamon Press, 1987)* mit einem in vivo abspaltbaren Rest substituiert ist. Beispiele für in vivo abspaltbare Reste sind Acylgruppen, Acyloxycarbonylgruppen, Alkoxycarbonylgruppen, Carbamoylgruppen, Phosphatreste. Ferner kommen auch die Disulfide, Isothiuroniumsalze, Thiosulfate (BUNTE-Salze) von Verbindungen der allgemeinen Formeln I, oder I(a) als in vivo in die freien Mercaptane überführbare Prodrugs in Betracht. Die erfindungsgemäßen Mercaptane der allgemeinen Formel I, insbesondere der Formel I(a), können ein oder mehrere Asymmetriezentren enthalten, was für das Vorkommen als Stereoisomere oder Racemate verantwortlich ist. Die erhaltenen Verbindungen der allgemeinen Formel I können gegebenenfalls nach an sich bekannten Methoden (vgl. z.B. *Allinger, N.L. und Elliel E.L. in "Topics in Stereochemistry" Vol. 6, Wiley Interscience, 1971)* in ihre Enantiomere und/ oder Diastereomere aufgetrennt werden.

Bevorzugte Verbindungen der Formel I(a) sind:
(R,S)-3-(2,3-dimercaptopropyl)pyrido[3,2-d]pyrimidin-2,4(1H,3H)-dion
(R,S)-3-(2,3-dimercapto-2-methyl-propyl)pyrido[ 3,2-d]pyrimidin-2,4(1H,3H)-dion
(R,S)-3-(3,4-dimercaptobutyl)pyrido[3,2-d]pyrimidin-2,4(1H,3H)-dion
(R,S)-3-(3,4-dimercapto-3-methyl-butyl)pyrido[3,2-d]pyrimidin-2,4(1H,3H)-dion
(R,S)-3-(2,3-dimercaptopropyl)pyrido[2,3-d]pyrimidin-2,4(1H,3H)-dion
(R,S)-3-(2,3-dimercapto-2-methyl-propyl)pyrido[2,3-d]pyrimidin-2,4(1H,3H)-dion
(R,S)-3-(3,4-dimercaptobutyl)pyrido[2,3-d]pyrimidin-2,4(1H,3H)-dion
(R,S)-3-(3,4-dimercapto-3-methyl-butyl)pyrido[2,3-d]pyrimidin-2,4(1H,3H)-dion
(R,S)-3-(2,3-dimercaptopropyl)-6,7-dimethylpyrido[2,3-d]pyrimidin-2,4(1H,3H)-dion
(R,S)-3-(2,3-dimercapto-2-methyl-propyl)-6,7-dimethylpyrido[ 2,3-d]pyrimidin-2,4(1 H,3H)-dion
(R,S)-3-(3,4-dimercaptobutyl)-6,7-dimethylpyrido[2,3-d]pyrimidin-2,4(1H,3H)-dion
(R,S)-3-(3,4-dimercapto-3-methyl-butyl)6,7-dimethylpyrido[2,3-d]pyrimidin-2,4(1 H,3H)-dion
(R,S)-3-(2,3-dimercaptopropyl)-6-pyridin-4-ylpyrido[ 2,3-d]pyrimidin-2,4(1 H,3H)-dion
(R,S)-3-(2,3-dimercapto-2-methyl-propyl) )-6-pyridin-4-yl pyrido[2,3-d]pyrimidin-2,4(1 H,3H)-dion
(R,S)-3-(3,4-dimercaptobutyl) )-6-pyridin-4-yl pyrido[ 2,3-d]pyrimidin-2,4(1H,3H)-dion
(R,S)-3-(3,4-dimercapto-3-methyl-butyl) )-6-pyridin-4-yl pyrido[ 2,3-d]pyrimidin-2,4(1 H,3H)-dion
(R,S)―3-(2,3-dimercaptopropyl)pteridin-2,4(1H,3H)-dion
(R,S)―3-(2,3-dimercapto-2-methyl-propyl)pteridin-2,4(1H,3H)-dion
(R,S)―3-(3,4-dimercaptobutyl)pteridin-2,4(1H,3H)-dion
(R,S)―3-(3,4-dimercapto-3-methyl-butyl)pteridin-2,4(1H,3H)-dion
(R,S)―3-(2,3-dimercaptopropyl)-6,7-dimethylpteridin-2,4(1H,3H)-dion
(R,S)―3-(2,3-dimercapto-2-methyl-propyl)-6,7-dimethylpteridin-2,4(1H,3H)-dion
(R,S)―3-(3,4-dimercaptobutyl)-6,7-dimethylpteridin-2,4(1 H,3H)-dion
(R,S)―3-(3,4-dimercapto-3-methyl-butyl)-6,7-dimethylpteridin-2,4(1H,3H)-dion
(R,S)-5-(2,3-dimercaptopropyl)-2H-pyrazolo[3,4-d]pyrimidin-4,6(5H,7H)-dion
(R,S)-5-(2,3-dimercapto-2-methyl-propyl)-2H-pyrazolo[3,4-d]pyrimidin-4,6(5H,7H)-dion
(R,S)-5-(3,4-dimercaptobutyl)-2H-pyrazolo[3,4-d]pyrimidin-4,6(5H,7H)-dion
(R,S)-5-(3,4-dimercapto-3-methyl-butyl)-2H-pyrazolo[3,4-d]pyrimidin-4,6(5H,7H)-dion
(R,S)-5-(2,3-dimercaptopropyl)-2-methyl-2H-pyrazolo[3,4-d]pyrimidin-4,6(5H,7H)-dion
(R,S)-5-(2,3-dimercapto-2-methyl-propyl)-2-methyl -2H-pyrazolo[3,4-d]pyrimidin-4,6(5H,7H)-dion
(R,S)-5-(3,4-dimercaptobutyl)-2-methyl -2H-pyrazolo[3,4-d]pyrimidin-4,6(5H,7H)-dion
(R,S)-5-(3,4-dimercapto-3-methyl-butyl)-2-methyl -2H-pyrazolo[3,4-d]pyrimidin-4,6(5H,7H)-dion
(R,S)-5-(2,3-dimercaptopropyl)-1H-pyrazolo[3,4-d]pyrimidin-4,6(5H,7H)-dion
(R,S)-5-(2,3-dimercapto-2-methyl-propyl)-1H-pyrazolo[3,4-d]pyrimidin-4,6(5H,7H)-dion
(R,S)-5-(3,4-dimercaptobutyl)-1H-pyrazolo[3,4-d]pyrimidin-4,6(5H,7H)-dion
(R,S)-5-(3,4-dimercapto-3-methyl-butyl)-1H-pyrazolo[3,4-d]pyrimidin-4,6(5H,7H)-dion
(R,S)-5-(2,3-dimercaptopropyl)-1-methyl-1H-pyrazolo[3,4-d]pyrimidin-4,6(5H,7H)-dion
(R,S)-5-(2,3-dimercapto-2-methyl-propyl)- 1-methyl -1H-pyrazolo[3,4-d]pyrimidin-4,6(5H,7H)-dion
(R,S)-5-(3,4-dimercaptobutyl)-1-methyl-1H-pyrazolo[3,4-d]pyrimidin-4,6(5H,7H)-dion
(R,S)-5-(3,4-dimercapto-3-methyl-butyl)-1-methyl-1H-pyrazolo[3,4-d]pyrimidin-4,6(5H,7H)-dion
(R,S)-3-(2,3-dimercaptopropyl)-5-methyl-1H-pyrrolo[3,2-d]pyrimidin-2,4(3H,5H)-dion
(R,S)-3-(2,3-dimercapto-2-methylpropyl)-5-methyl-1H-pyrrolo[3,2-d]pyrimidin-2,4(3H,5H)-dion
(R,S)-3-(3,4-dimercaptobutyl)-5-methyl-1H-pyrrolo[3,2-d]pyrimidin-2,4(3H,5H)-dion
(R,S)-3-(3,4-dimercapto-3-methylbutyl)-5-methyl-1H-pyrrolo[3,2-d]pyrimidin-2,4(3H,5H)-dion
(R,S)-3-(2,3-dimercaptopropyl)-5,7-dimethyl-1H-pyrrolo[3,2-d]pyrimidin-2,4(3H,5H)-dion
(R,S)-3-(2,3-dimercapto-2-methylpropyl)-5,7-dimethyl-1H-pyrrolo[3,2-d]pyrimidin-2,4(3H,5H)-dion
(R,S)-3-(3,4-dimercaptobutyl)-5,7-dimethyl-1H-pyrrolo[3,2-d]pyrimidin-2,4(3H,5H)-dion
(R,S)-3-(3,4-dimercapto-3-methylbutyl)-5,7-dimethyl-1H-pyrrolo[3,2-d]pyrimidin-2,4(3H,5H)-dion
(R,S)-3-(2,3-dimercaptopropyl)-5,6,7-trimethyl-1H-pyrrolo[3,2-d]pyrimidin-2,4(3H,5H)-dion
(R,S)-3-(2,3-dimercapto-2-methylpropyl)-5,6,7-trimethyl-1H-pyrrolo[3,2-d]pyrimidin-2,4(3H,5H)-dion
(R,S)-3-(3,4-dimercaptobutyl)-5,6,7-trimethyl-1H-pyrrolo[3,2-d]pyrimidin-2,4(3H,5H)-dion
(R,S)-3-(3,4-dimercapto-3-methylbutyl)-5,6,7-trimethyl-1H-pyrrolo[3,2-d]pyrimidin-2,4(3H,5H)-dion
(R,S)-3-(2,3-dimercaptopropyl)furo[2,3-d]pyrimidin-2,4(1H,3H)-dion
(R,S)-3-(2,3-dimercapto-2-methylpropyl)furo[2,3-d]pyrimidin-2,4(1H,3H)-dion
(R,S)-3-(3,4-dimercaptobutyl)furo[2,3-d]pyrimidin-2,4(1H,3H)-dion
(R,S)-3-(3,4-dimercapto-3-methyl-butyl)furo[2,3-d]pyrimidin-2,4(1H,3H)-dion
(R,S)-3-(2,3-dimercaptopropyl)-1-methyl-thieno[3,2-d]pyrimidin-2,4(1H,3H)-dion
(R,S)-3-(2,3-dimercapto-2-methylpropyl)-1-methyl-thieno[3,2-d]pyrimidin-2,4(1H,3H)-dion
(R,S)-3-(3,4-dimercaptobutyl)-1-methyl-thieno[3,2-d]pyrimidin-2,4(1H,3H)-dion
(R,S)-3-(3,4-dimercapto-3-methylbutyl)-1-methyl-thieno[3,2-d]pyrimidin-2,4(1 H,3H)-dion
(R,S)-3-(2,3-dimercaptopropyl)-1-methyl-thieno[2,3-d]pyrimidin-2,4(1H,3H)-dion
(R,S)-3-(2,3-dimercapto-2-methylpropyl)-1-methyl-thieno[2,3-d]pyrimidin-2,4(1H,3H)-dion
(R,S)-3-(3,4-dimercaptobutyl)-1-methyl-thieno[2,3-d]pyrimidin-2,4(1H,3H)-dion
(R,S)-3-(3,4-dimercapto-3-methylbutyl)-1-methyl-thieno[2,3-d]pyrimidin-2,4(1 H,3H)-dion
(R,S)-3-(2,3-dimercaptopropyl)-1-methylchinazolin-2,4(1H,3H)-dion
(R,S)-3-(2,3-dimercapto-2-methylpropyl)-1-methylchinazolin-2,4(1H,3H)-dion
(R,S)-3-(3,4-dimercaptobutyl)-1-methylchinazolin-2,4(1H,3H)-dion
(R,S)-3-(3,4-dimercapto-3-methylbutyl)-1-methylchinazolin-2,4(1 H,3H)-dion

Besonders bevorzugte Verbindungen der Formel I(a) sind:
(R,S)-3-(2,3-Dimercaptopropyl)chinazolin-2,4-(1H,3H)dion
(R,S)-3-(2,3-Dimercapto-2-methylpropyl)chinazolin-2,4-(1 H,3H)dion
(R,S)-7-Chlor-3-(2,3-dimercaptopropyl)chinazolin-2,4-(1H,3H)dion
(R,S)-6-Chlor-3-(2,3-dimercaptopropyl)chinazolin-2,4-(1 H,3H)dion
(R,S)-3-(2,3-Dimercaptopropyl)-5-methyl-chinazolin-2,4-(1H,3H)dion
(R,S)-3-(2,3-Dimercaptopropyl)-8-methyl-chinazolin-2,4-(1H,3H)dion
(R,S)-3-(2,3-Dimercaptopropyl)-6,7-dimethoxy-chinazolin-2,4-(1H,3H)dion
(R,S)-N-Benzyl-3-(2,3-dimercaptopropyl)-2,4-dioxo-1,2,3,4-tetrahydrochinazolin-7-carboxamide
(R,S)-3-(2,3-dimercaptopropyl)pyrido[3,2-d]pyrimidin-2,4(1H,3H)-dion
(R,S)-3-(2,3-dimercapto-2-methyl-propyl)pyrido[3,2-d]pyrimidin-2,4(1H,3H)-dion
(R,S)-3-(2,3-dimercaptopropyl)pyrido[2,3-d]pyrimidin-2,4(1H,3H)-dion
(R,S)-3-(2,3-dimercapto-2-methyl-propyl)pyrido[2,3-d]pyrimidin-2,4(1H,3H)-dion
(R,S)―3-(2,3-dimercaptopropyl)pteridin-2,4(1H,3H)-dion
(R,S)―3-(2,3-dimercapto-2-methyl-propyl)pteridin-2,4(1H,3H)-dion
(R,S)-5-(2,3-dimercaptopropyl)-2H-pyrazolo[3,4-d]pyrimidin-4,6(5H,7H)-dion
(R,S)-5-(2,3-dimercapto-2-methyl-propyl)-2H-pyrazolo[3,4-d]pyrimidin-4,6(5H,7H)-dion
(R,S)-5-(2,3-dimercaptopropyl)-1H-pyrazolo[3,4-d]pyrimidin-4,6(5H,7H)-dion
(R,S)-5-(2,3-dimercapto-2-methyl-propyl)-1H-pyrazolo[3,4-d]pyrimidin-4,6(5H,7H)-dion
(R,S)-3-(2,3-dimercaptopropyl)-5-methyl-1H-pyrrolo[3,2-d]pyrimidin-2,4(3H,5H)-dion
(R,S)-3-(2,3-dimercapto-2-methylpropyl)-5-methyl-1H-pyrrolo[3,2-d]pyrimidin-2,4(3H,5H)-dion
(R,S)-3-(2,3-dimercaptopropyl)-1-methylchinazolin-2,4(1H,3H)-dion
(R,S)-3-(2,3-dimercapto-2-methylpropyl)-1-methylchinazolin-2,4(1H,3H)-dion
(R,S)-3-(2,3-Dimercaptopropyl)thieno[3,2-d]pyrimidin-2,4(1H,3H)-dion
(R,S)-3-(2,3-Dimercaptopropyl)-5,6,7,8-tetrahydro[1]benzothieno[2,3-d]pyrimidin-2,4(1 H,3H)-dion
(R,S)-3-(2,3-Dimercaptopropyl)-6-phenylthieno[2,3-d]pyrimidin-2,4(1H,3H)-dion

Die erfindungsgemässen Dimercaptane sind zugänglich durch saure oder alkalische Hydrolyse von Monomercaptanen der allgemeinen Formel II. 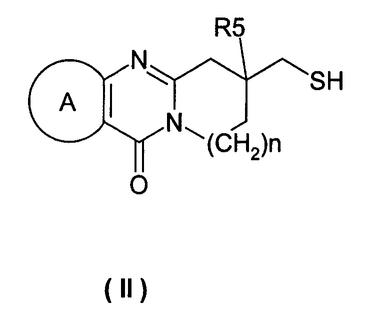
n = die Zahlen 0 oder 1,
A = ein anellierter Benzolring oder ein anellierter fünf- bis siebengliedriger Cycloalkylring oder ein anellierter fünf- bis siebengliedriger Cycloheteroalkylring mit mindestens einem Sauerstoff- oder Schwefelatom oder ein über zwei Kohlenstoffatome gebundener fünf- bis siebengliedriger Heteroaromat mit einem, zwei oder drei Stickstoff-, Sauerstoff- und/oder Schwefelatomen als Heteroatomen, wobei A unsubstituiert, mono-, di- oder trisubstituiert ist mit Halogen, C1-C6 Alkyl, C1-C3- Alkylthio-, C6-C12-Aryl-, Nitro-, Carbamoyl-, C1-C4 Alkoxy, -CN, CF₃, NH₂ -, Carboxy-, C1-C4 Alkoxycarbonyl, C1-C4 N-alkyl-carbamoyl oder C1-C5 Alkenyl
R5 = Wasserstoff, Methyl

Monomercaptane der allgemeinen Formel II sind für das Chinazolinsystem (A= Benzo) bereits bekannt (DD 100 46 728).

Monomercaptane der allgemeinen Formel II mit heterocyclisch kondensiertem Pyrimidingrundkörper (A= Heterocyclus) wurden bereits vorgeschlagen.

Der Aufbau der Vorstufen II mit A= Heterocyclus erfolgt dabei in der Regel nach den aus der Chemie der entsprechenden Chinazoline hinlänglich bekannten Mustern.
Die Ausgangsverbindungen bzw. Zwischenstufen in Gestalt der entsprechenden heterocyclischen Anthranilsäureanaloga sind teilweise kommerziell erhältlich, teilweise literaturbekannt oder dem Fachmann nach literaturbekannten Verfahren zugänglich. Als zusammenfassende Literatur siehe z.B.: *Methoden der Organischen Chemie (Houben Weyl) Thieme Verlag, Stuttgart, Bd. E 8b (1994) S. 1 ff. und Bd. E 8a (1993) S. 668 ff. (Thiazole); Bd. E 6a (1994) S. 556 ff. (Pyrrole); Bd. E 8b (1994) S. 399 ff. (Pyrazole); Bd. E 6 (1994) S. 186 ff. (Thiophene); Bd. 9b*/*I (1998) S. 8 ff. (Pyrimidine); Bd. 9b*/*I (1998) S. 250 ff. (Pyrazine); O. Meth-Cohn (Ed.), Comprehensive heterocyclic Chemistry, Pergamon Press, Oxford, 1996; The Chemistry of heterocyclic Compounds, Wiley Interscience, New York, Bd. I (1950) u. ff.* Als Beispiele für spezielle Zugänge zu Synthesebausteinen vom Typ heterocyclischer β-Enaminocarbonsäuredeivate seien genannt: *R. W. Sabnis,* The Gewald-Synthesis, Sulfur Reports 16 (1994) S. 1 ff.(Thiophene); R.W. Sabnis, D.W. Ragnekar, N.D. Sonawane, 2-Aminothiophenes by the Gewald Reaction, J. Heterocyclic Chem. 36 (1999) 333 ff. (Thiophene); Briel, D., Maschke, T., Wagner, G., Synthese von Thieno[3,3-d]- und [3,4-d]pyrimidinen durch alternative Ringschlußreaktionen, Pharmazie 47 (1992) 577 ff. (Thiophene); Pnto, I.L., Jarvest, R.L., Serafinowska, H.T., The Syntesis of 5-Alkoxy and 5-Amino Substituted Thiophenes, Tetrahedron Lett. 41 (2000) 1597- 1699, Dave, C.G., Synthesis and Reactions of Fluoroaryl Substituted 2-Amino-3-cyanopyrroles and Pyrrolo[2,3-d]pyrimidines, J. Heterocyclic Chem. 36 (1999) 729 ff. (Pyrrole); Lim,M., Klein, R.S., Fox, J.J., A New Synthesis of Pyrrolo[3,2-d]pyrimidines via 3-Amino-2-carboalkoxypyrroles, J. Org. Chem. 44 (1979) 3826 ff. (Pyrrole); Hanning, E., Beyer, G., Laban, G., Pharmazie 41 (1986) 381 ff. (Pyrazole); Bridson, P.K., Wang, X., 1-Substituted Xanthines, Synthesis 1995 S. 855 ff. (Imidazole) sowie WO 99/11631 (Thiophene) und US 4,835,157 (Thiophen, Furan).

Daneben sind auch verschiedene Synthesen für heterocyclisch anellierte Pyrimidine bekannt, bei denen der Aufbau des mehrcyclischen Grundsystems ausgehend von entsprechend substituierten Pyrimidinen erfolgt, z.B.: EI-Ahl, A.A.S. u.a., A One-Pot Synthesis of Pyrido[2,3-d]- and Quinolino[2,3-d]pyrimidines, Heterocycles 55 (2001) 1315 ff.; Falch, E., Synthesis and Structure Determination of Thiazolo- and Thiazinopteridinones, Acta Chemica Scandinavica B 31 (1977) 167 ff.; Cottam, H.B. u.a., Substituted Xanthines, Pteridinediones and Related Compounds as Potential Antiinflammatory Agents. Synthesis and Biological Evaluation of Inhibitors of Tumor Necrosis Factor α, J. Med. Chem. 39 (1996) 2 ff.; Bhuyan, Studies on Uracils: A Facile One-Pot Synthesis of Pyrazolo[3,4-d]pyrimidines, Tetrahedron Lett. 43 (2002), WO 00/00491 (Thiadiazole, Imidazole, Pyrazine).

Es ist bereits bekannt, durch säure- oder basenkatalysierten Ringöffnung von Thiazolo- bzw. Thiazinopyrimidinen zu den entsprechenden Mercaptanen zu gelangen (z.B.: *Falch,E., Synthesis and Structure Determination of Thiazolo- and Thiazinopteridones, Acta Chem. Scand. B 31 (1977) 167 ff; Gütschow,M.; Drössler.K.; Leistner,S., Polycyclic Azines with Heteroatoms in the 1- and 3-Position. 40. Synthesis of Heterocyclic Immunomodulators. 2. 3-Mercaptolakylthieno(2,3-d)pyrimidine-2,4(1H,-3H)--diones: Synthesis and Testing of Immunostimulant Activity, Aren Pharm 328:3 (1995) 231 ff*). Bisher wurde nicht versucht, die katalytische Ringöffnung auf Pyrimidindione der allgemeinen Formel II anzuwenden. Überraschenderweise gelingt die Ringöffnung aber unter Bedingungen, die dem Substituentencharakter angepasst sind. Die nachstehend aufgeschriebene allgemeine Arbeitsvorschrift gibt die Methodik wieder, nach welcher sich der Ring öffnen lässt, während die Beispiele einen besonders günstigen Syntheseweg zu den erfindungsgemäßen Pyrimidindionen weisen.

Die Substitution von N1 (Rest R4 in Formel (I) und (la)) erfolgt bevorzugt durch nachträgliche Alkylierung des entsprechend geschützten erhaltenen tricylischen Mercaptans, Geeignete Verfahren für diese nachträgliche Alkylierung sind dem Fachmann ausreichend bekannt.

Bezüglich der hier beschriebenen Verbindungen sind die zugrundeliegenden Struktur-Wirkungs-Beziehungen noch unklar und die gefundenen enzyminhibitorischen Eigenschaften der Verbindungen daher als neu und überraschend anzusehen.

Allgemeine Arbeitsvorschrift zur Darstellung der erfindungsgemäßen Dimercaptane:

Saure Hydrolyse (Variante A)
5 mmol Mercaptomethylvorstufe (allg. Formel II) werden in eine aus 0,6 ml konz. Schwefelsäure, 1,5 ml Eisessig und 66 ml Wasser bereitete Säuremischung eingetragen und am Rückfluß unter chromatografischer Kontrolle (DC oder HPLC) bis zum vollständigen Umsatz erhitzt. Die durchschnittliche Reaktionszeit beträgt 8- 10 Stunden. Der nach dem Erkalten (evtl. mit Wasser verdünnen) ausgefallene Niederschlag wird abgetrennt, mit Wasser gewaschen, getrocknet und ggf. umkristallisiert.

Alkalische Hydrolyse (Variante B)
5 mmol Mercaptomethylvorstufe (allg. Formel II) werden unter Stickstoff in 50 ml einer Mischung aus 3N NaOH (stickstoffgesättigt)/ Ethanol (10:1 ) und 100 mg Zinkstaub eingetragen. Der Ansatz wird unter kontinuierlichem Einleiten von Stickstoff bei 80 bis 85 °C bis zum vollständigen Umsatz (DC- oder HPLC-Kontrolle) gerührt. Die durchschnittliche Reaktionszeit beträgt 5-8 Stunden. Nach beendeter Reaktion wird der erkaltete Reaktionsansatz bei direkter Eiskühlung unter starkem Rühren und Einleiten von Stickstoff in 10%ige Salzsäure filtriert. Der Niederschlag wird nach Aufbewahrung über Nacht bei 4°C abgesaugt, mit Wasser gewaschen, getrocknet und ggf. umkristallisiert.

Die Herstellung der N1-substituierten Verbindungen (R4 ≠ H) erfolgt nach literaturbekannten Verfahren, z.B. durch Alkylierung der geschützten Dimercaptane mit einem entsprechenden Alkylhalogenid / K₂CO₃ / DMF.

Bei der Synthese der Verbindungen der allg. Formeln (I) oder (la) ggf. zu verwendende Schutzgruppenchemie ist dem Fachmann ausreichend bekannt und wird von diesem beherrscht.

Im Rahmen der Erfindung ist es gelungen, Substanzklassen zu erschließen, deren Vertreter die gewünschte Wirkung zeigen und die darüber hinaus synthetisch leicht zugänglich sind, die in Substanz und in Lösung stabil sind, gut handhabbar, zu möglichen Arzneimitteln formuliert werden können. Durch Testung der neuen Verbindungen der allgemeinen Formel I, insbesondere der Formel I(a), an unterschiedlichen humanen MMPs (MMP-2, rekombinante katalytische Domäne der MMP-3, rekombinante katalytische Domäne der MMP-8, MMP-9, rekombinante katalytische Domäne der MMP-14) werden die Wirksamkeit und Spezifitäten der erfindungsgemäßen Verbindungen belegt. Spezifische Inhibitoren würden besonders beim Einsatz als Arzneimittel nur die gewünschten Zielenzyme beeinflussen und nicht das ausbalancierte Zusammenspiel der anderen MMPs stören und somit mögliche unerwünschte Nebenwirkungen vermeiden helfen.

Die Erfindung wird nachfolgend anhand der Beispiele näher beschrieben.

### Beispiel 1

### (R,S)-3-(2,3-Dimercaptopropyl)chinazolin-2,4-(1H,3H)dion (allg. Formel (la), A= Benzo, n= 0, R4, R5 = H)

Die Zielverbindung wurde aus (R,S)-2-Mercaptomethyl-2,3-dihydro-5H-thiazolo[2,3-b]chinazolin-5-on (allg. Formel II, A= Benzo; n= 0; R5 = H, H; DD 100 46 728) nach Variante B erhalten.
Ausbeute 58 %
Farbloser Feststoff
F.: 181- 185 °C
C₁₁H₁₂N₂O₂S₂ (268)
MS m/e (%B): 268 (2, M⁺ ), 234 (41), 201 (70), 163 (100), 146 (85)

### Beispiel 2

### (R,S)-3-(2,3-Dimercapto-2-methylpropyl)chinazolin-2,4-(1H,3H)dion (allg. Formel (la), A= Benzo, n= 0, R4= H, R5= Methyl)

Die Zielverbindung wurde aus (R,S)-2-Mercaptomethyl-2-methyl-2,3-dihydro-5Hthiazolo[2,3-b]chinazolin-5-on (allg. Formel II, A= Benzo, n= 0, R5= Methyl; DD 100 46 728) nach Variante B erhalten.
Ausbeute 45 %
Farbloser Feststoff
F.: 128- 131 °C
C₁₂H₁₄N₂O₂S₂ (282)
MS m/e (%B): M⁺ 282 (2, M⁺), 217 (8), 201 (18), 137 (22), 119 (76), 43 (100)

### Beispiel 3

### (R,S)-7-Chlor-3-(2,3-dimercaptopropyl)chinazolin-2,4-(1H,3H)dion (allg. Formel (la), A= Benzo mit 7-Chlor, n= 0, R4, R5= H)

Die Zielverbindung wurde aus (R,S)-8-Chlor-2-mercaptomethyl-2,3-dihydro-5Hthiazolo[2,3-b]chinazolin-5-on (allg. Formel II, A= Benzo mit 8-Chlor, n= 0, R5= H; DD 100 46 728) nach Variante B erhalten.
Ausbeute 70 %
Farbloser Feststoff
F.: 183- 186 °C
C₁₁H₁₁ClN₂O₂S₂ (302,9)
MS m/e (%B): 302 (1, M⁺), 268 (29), 235 (75), 197 (100), 180 (95)

### Beispiel 4

### (R,S)-6-Chlor-3-(2,3-dimercaptopropyl)chinazolin-2,4-(1H,3H)dion (allg. Formel (la), A= Benzo mit 6-Chlor, n= 0, R4, R5= H)

Die Zielverbindung wurde aus (R,S)-7-Chlor-2-mercaptomethyl-2,3-dihydro-5Hthiazolo[2,3-b]chinazolin-5-on (allg. Formel II, A= Benzo mit 7-Chlor, n= 0, R5= H; F. 162- 166 °C), was analog der Vorstufe von Beispiel 3 zugänglich ist, nach Variante B erhalten.
Ausbeute 76 %
Farbloser Feststoff
F.: 212- 218 °C
C₁₁H₁₁ClN₂O₂S₂ (302,9)
MS m/e (%B): 268 (5), 236 (80), 221 (100), 196 (35), 180 (90)

### Beispiel 5

### (R,S)-3-(2,3-Dimercaptopropyl)-5-methyl-chinazolin-2,4-(1H,3H)dion (allg. Formel (la), A= Benzo mit 5-Methyl, n= 0, R4, R5= H)

Die Zielverbindung wurde aus (R,S)-2-mercaptomethyl-6-methyl-2,3-dihydro-5Hthiazolo[2,3-b]chinazolin-5-on (allg. Formel II, A= Benzo mit R6= 6-Methyl, n= 0, R5= H;; F. 203 °C), was analog der Vorstufe von Beispiel 1 zugänglich ist, nach Variante B erhalten.
Ausbeute 65 %
Farbloser Feststoff
F.: 176- 178 °C
C₁₂H₁₄N₂O₂S₂ (282,5)
MS m/e (%B): 282 (1, M⁺), 264 (70), 217 (100), 177 (28)

### Beispiel 6

### (R,S)-3-(2,3-Dimercaptopropyl)-8-methyl-chinazolin-2,4-(1H,3H)dion (allg. Formel (la), A= Benzo mit 8-Methyl, n= 0, R4, R5= H)

Die Zielverbindung wurde aus (R,S)-2-Mercaptomethyl-9-methyl-2,3-dihydro-5Hthiazolo[2,3-b]chinazolin-5-on (allg. Formel II, A= Benzo mit 9-Methyl, n= 0, R5= H; F. 180- 184 °C), was analog der Vorstufe von Beispiel 1 zugänglich ist, nach Variante B erhalten.
Ausbeute 52 %
Farbloser Feststoff
F.: 169- 171 °C
C₁₂H₁₄N₂O₂S₂ (282,5)
MS m/e (%B) 282 (10,M⁺); 249 (40); 192 (10); 177 (100); 161 (20)

### Beispiel 7

### (R,S)-3-(2,3-Dimercaptopropyl)-6,7-dimethoxy-chinazolin-2,4-(1H,3H)dion (allg. Formel I (a), A= Benzo mit 6,7-Dimethoxy, n= 0, R4, R5= H)

Die Zielverbindung wurde aus (R,S)-2-Mercaptomethyl-7,8-dimethoxy-2,3-dihydro-5H-thiazolo[2,3-b]chinazolin-5-on (allg. Formel II, A= Benzo mit 7,8-Dimethoxy, n= 0, R5= H; 180- 184 °C), was analog der Vorstufe von Beispiel 1 zugänglich ist, nach Variante A erhalten.
Ausbeute 79 %
Farbloser Feststoff
F.: 197- 204 °C
C₁₃H₁₆N₂O₄S₂ (328,5)
MS m/e (%B): 328 (30, M⁺), 295 (55), 261 (70), 223 (100)

### Beispiel 8

### (R,S)-N-Benzyl-3-(2,3-dimercaptopropyl)-2,4-dioxo-1,2,3,4-tetrahydrochinazolin-7-carboxamide (allg. Formel (la), A= Benzo mit 7-N-Benzylcarboxamid, n= 0, R4, R5= H)

Die Zielverbindung wurde aus (R,S)-N-Benzyl-2-mercaptomethyl-5-oxo-2,3-dihydro-5H-thiazolo[2,3-b]chinazolin-8-carboxamid (allg. Formel II, A= 8-N-Benzylcarboxamid, n= 0, R5= H; F. 174- 176 °C), was analog der Vorstufe von Beispiel 1 zugänglich ist, nach Variante A erhalten.
Ausbeute 19,5 %
Farbloser Feststoff
F.: 214- 218 °C (Methanol)
C₁₉H₁₉N₃O₃S₂ (401,5)
MS m/e (%B): 368 (8), 335 (35), 295 (20), 216 (55)

### Beispiel 9

### (R,S)-3-(2,3-Dimercaptopropyl)thieno[3,2-d]pyrimidin-2,4(1H,3H)-dion (allg. Formel (la), A= Thieno, n= 0, R4, R5= H)

Die Zielverbindung wurde aus dem bereits von uns vorgeschlagenen (R,S)-6-Mercaptomethyl-6,7-dihydro-thiazolo[3,2-a]thieno[3,2-d]pyrimidin-9-on (allg. Formel II, A= Thieno, n=0, R5=H ) nach Variante B erhalten.
Ausbeute 68 %
Schwach gelber Feststoff
F.: 170- 174 °C
C₉H₁₀N₂O₂S₃ (274)
MS m/e (%B): 274 (3, M⁺), 240 (45), 207 (70), 169 (97), 152 (98), 126 (100)

### Beispiel 10

### (R,S)-3-(2,3-Dimercaptopropyl)-5,6,7,8-tetrahydro[1]benzothieno[2,3-d]pyrimidin-2,4(1H,3H)-dion (allg. Formel (la), A= Thieno mit R6= anellierter Cyclohexylring, n= 0, R4, R5= H)

Die Zielverbindung wurde aus dem bereits von uns vorgeschlagenen (R,S)-2-Mercaptomethyl-2,3,6,7,8,9-hexahydro-5H-benzo[4,5]thieno[2,3-d]thiazolo[3,2-a]pyrimidin-5-on (allg. Formel II, A= Thieno mit R6= anellierter Cyclohexylring, n=0, R5= H ) nach Variante B erhalten.
Ausbeute 56 %
Cremefarbener Feststoff
F.: 97- 100 °C
C₁₃H₁₆N₂O₂S₃ (328)
MS m/e (%B): 328 (2, M⁺), 310 (100), 282 (40), 263 (27), 235 (20)

### Beispiel 11

### (R,S)-3-(2,3-Dimercaptopropyl)-6-phenylthieno[2,3-d]pyrimidin-2,4(1H,3H)-dion (allg. Formel (la), A= Thieno mit R6= 6-Phenyl, n= 0, R4, R5= H)

Die Zielverbindung wurde aus dem bereits von uns vorgeschlagenen (R,S)-2-Mercaptomethyl-7-phenyl-2,3-dihydro-5H-thiazolo[3,2-a]thieno[2,3-d]pyrimidin-5-on (allg. Formel II, A= Thieno mit 7-Phenyl, n=0, R5=H ) nach Variante B erhalten.
Ausbeute 47 %
Hellbeiges Pulver
F.: 197- 205 °C (Chloroform/ Ethanol/ Heptan)
C₁₅H₁₄N₂O₂S₃ (350,5)
S m/e (%B): 350 (10, M⁺), 324 (40), 317 (10), 283 (15), 245 (20), 202 (100)

### Beispiel 12

### - Präparation von Matrix-Metalloproteinase-2 (MMP-2)

MMP-2 (Gelatinase) wird von kultivierten dermalen Fibroblasten in beträchtlichen Mengen in das Kulturmedium abgegeben und ist daher leicht zugänglich. Die sezernierte und inaktive Proform des Enzyms wird durch Behandlung mit organischen Quecksilberverbindungen in die enzymatisch aktive Form überführt.

Hierzu werden humane dermale Fibroblasten nach etablierten Standardmethoden gewonnen und kultiviert. Aus dem konditionierten Zellmedium wird humane MMP-2 durch folgende Methode gewonnen:
500 ml Zellmedium werden 30 min. bei 10 000 g zentrifugiert und der Überstand mit einem Vol. 2-fach Bindungspuffer (40 mM Tris-HCI pH 7,5 , 10 mM CaCl₂, 1 M NaCI, 0,2% (v/v) Triton X-100) versetzt. Dazu gibt man 6 ml Gelatine-Agarose, welche mit Bindungspuffer äquilibriert wurde, und schüttelt für 1 Std. auf Eis. Die beladene Gelatine-Agarose wird in eine Säule überführt und mit mindestens 10 Vol. Bindungspuffer proteinfrei gespült. Die Elution der gebundenen MMP-2 erfolgt mit 2 Gelvolumen Bindungspuffer plus 5% (v/v) DMSO. Das Eluat enthält die humane MMP-2 als inaktives Zymogen in stark angereicherter Form. Fremdproteine sind TIMP-2 und Fibronektin; das letztere kann durch eine Gelfiltration an Sephacryl S-200 abgetrennt werden. Laufpuffer ist 100 mM Tris-HCI pH 7,5, 10 mM CaCl₂, 100 mM NaCI, 0,05% (v/v) Brij 35.
Die inaktive humane MMP-2 wird durch zweistündige Inkubation bei 37 °C in Gegenwart von 1 mM p-Aminophenylmercuric Acetate (APMA) aktiviert. Zur Beseitigung von APMA nach Aktivierung wird die Probe an Sephadex G-25 in Laufpuffer (s.o.) filtriert.

### - Präparation der humanen Kollagenase MMP-9

Native MMP-9 wurde reproduzierbar und in guter Ausbeute und Reinheit aus humanem Buffy Coat gewonnen. Hierzu wird Buffy Coat mit 10% (v/v) Triton X-100 auf eine Endkonzentration von 0,4% gebracht, 30 min. auf Eis geschüttelt und dann 1 Vol. 2-fach Bindungspuffer (40 mM Tris-HCI pH 7,5, 10 mM CaCl₂, 1 M NaCI, 0,2% (v/v) Triton X-100) hinzugefügt und weitere 30 min. auf Eis geschüttelt. Die Lösung wird 15 min. bei 16 000 Upm an einem SS-34 Rotor bei 4°C abzentrifugiert und über Glaswolle filtriert. Das Filtrat wird mit Gelatine-Agarose, welche mit Bindungspuffer äquilibriert wurde, gebatcht und 1 Std. auf Eis geschüttelt.
Die beladene Gelatine-Agarose wird in eine Säule überführt und mit mindestens 10 Vol. Bindungspuffer proteinfrei gespült. Die Elution der gebundenen MMP-9 erfolgt mit 2 Gelvolumen Bindungspuffer plus 5% (v/v) DMSO.
Das Eluat kann zum Puffer-Austausch und gleichzeitiger Abtrennung geringer Verunreinigungen an MMP-2 über eine Gelfiltration an Sephadex G-75 aufgetrennt werden. Hierzu wird der Puffer I (20 mM Tris-HCI pH 7,5, 5 mM CaCl₂, 100 mM NaCI, 0,1% (v/v) Triton X-100) verwendet.
Das so gewonnene Eluat enthällt MMP-9 in den drei bekannten Zustandsformen: Monomer, Homodimer, Heterodimer. Die Reinheit des Enzyms beträgt ca. 90%, wobei die restlichen Fremdproteine Fibronektin und äußerst geringe Mengen an TIMP's sind.
Das latente Enzym wird durch 30 - 60 min. Inkubation bei 37°C mit 1/100 Vol. Trypsin (10 mg/ml) aktiviert. Trypsin wird durch Zugabe von 1 mM PMSF oder mit einem spezifischen Inhibitor (TKI) gehemmt.

### - Klonierung und Expression der katalytischen Domäne der MMP-8

Die Nutzung der katalytischen Domäne der MMP-8 als weiteres Testenzym wurde deshalb gewählt, weil es eine hohe Stabilität besitzt und darüber hinaus als aktives Enzym vorliegt. Es muß deshalb nicht aktiviert werden, was eine der häufigsten Fehlerursachen darstellt, da die zur Aktivierung genutzten Quecksilberverbindungen häufig mit dem Testsystem bzw. dem Enzym interferieren und dadurch die Messergebnisse verfälschen werden. Die Klonierungsstrategie gestalteten wir so, dass wir statt des Gesamtenzyms nur dessen enzymatisch aktive katalytische Domäne in E. coli klonierten. Mit der konstruierten katalytischen Domäne der MMP-8 erzielten wir ein stabiles, enzymatisch aktives und hochreines Enzym, welches für die Routineuntersuchungen der inhibitorischen Aktivität der synthetisierten Inhibitoren sehr gut geeignet war und die Messergebnisse der einzelnen Messserien miteinander vergleichbar machte.
Die Klonierung und Expression der katalytischen Domäne der MMP-8 wurde entsprechend den Angaben von SCHNIERER et al. (Schnierer S, Kleine T, Gote T, Hillemann A, Knäuper V, Tschesche H: The recombinant catalytic domain of human neutrophil collagenase lacks type I collagen substrate specificity. Biochem Biophys Res Comm (1993) vol. 191 No. 2, 319-326) durchgeführt.

### - Klonierung und Expression der katalytischen Domäne der MMP-3

Die katalytische Domäne der humanen MMP-3 wurde nach den Angaben in folgender Publikation kloniert:
Ye Q-Z, Johnson LL, Hupe DJ, Baragi V: Purification and Characterization of the Human Stromelysin Catalytic Domain Expressed in Escherichia coli. Biochemistry (1992), 31, 11231-11235).
Nach Rückfaltung des Enzyms und Gelfiltration an Sephadex G-50 (Laufpuffer 100 mM Tris-HCI pH 7,5, 10 mM CaCl₂, 100 mM NaCI, 0,05% Brij 35) erhielten wir ein zu 95% reines Präparat.

### - Klonierung und Expression der katalytischen Domäne der MT1-MMP

Die katalytische Domäne der humanen MT1-MMP wurde nach den Angaben in folgender Publikation kloniert:
Lichte A, Kolkenbrock H, Tschesche H: The recombinant catalytic domain of membrane-type matrix metalloproteinase-1 (MT1-MMP) induces activation of progelatinase A and progelatinase A complexed with TIMP-2. FEBS letters (1996), 397, 277-282.

Nach Rückfaltung des Enyzms, Autoprozessierung zur aktiven Form und Gelfiltration an Sephadex G-50 (Laufpuffer 20 mM Tris-HCI pH 7,5, 5 mM CaCl₂, 100 mM NaCI, 0,1 mM ZnCl₂, 0,02% (w/v) Natriumazid) erhielten wir ein zu 95% reines Präparat.

### - Quantitativer Fluoreszenz-Assay für Matrix-Metalloproteinasen

Durch enzymatische Spaltung des synthetischen Substrates Mca-Pro-Leu-Gly-Leu-Dpa-Ala-Arg-NH₂ durch die jeweilige Kollagenase, wird die fluoreszente Gruppe Mca ((7-Methoxycoumarin-4-yl)acetyl) vom internen Quencher Dpa ((N-3-(2,4-dinitrophenyl)-L-2,3-diaminopropienyl) getrennt. Dabei erfolgt eine starke Zunahme der Fluoreszenz im Messansatz, die am Fluorimeter quantifiziert werden kann (λₑₓ 328 nm, λₑₘ 393 nm) und innerhalb der ersten Minuten linear verläuft. Eine gewisse Spezifität des Tests für Matrix-Metalloproteinasen ergibt sich einerseits aus der Aminosäuresequenz -Pro-Leu-Gly-Leu- im Substrat und andererseits durch die gewählten Inkubationsbedingungen. Matrix-Metalloproteinasen spalten das Substrat an der Bindung Gly-Leu. Gemessen wird die proteolytische Restaktivität vorinkubierter Ansätze von Enzym und Inhibitor, wobei die Substrat- und Enzymkonzentration konstant gehalten und die Inhibitorkonzentration variiert wurde. Es wurden für jeden getesteten Inhibitor drei Messreihen mit unterschiedlicher Substratkonzentration aufgenommen. Aus der zeitabhängigen Fluoreszenzzunahme wird die Enzymaktivität in Fluoreszenzeinheiten pro min berechnet. Die Bestimmung der Kᵢ-Werte erfolgte graphisch nach der Methode von DIXON (1953) durch Auftragung der reziproken Reaktionsgeschwindigkeit 1/v (y-Achse) gegen Inhibitorkonzentration (x-Achse).

### Assay

1984 µl Messpuffer (100mM Tris-HCI pH 7,5, 100 mM NaCI, 10 mM CaCl₂, 0,05% Brij 35 bzw. 50 mM MES-NaOH, pH 6.0, 10 mM CaCl2, 100 mM NaCI, 0.05% Brij 35 im Fall der KaDo MMP-3)
2 µl Inhibitor, gelöst in DMSO, bzw. DMSO allein (nichtinhibierender Ansatz) 4 µl Enzym
- 5 min. Vorinkubation des Enzym-Inhibitor-Gemischs bei Raumtemperatur unter Rühren
- Start der Reaktion mit 10 µl Substrat gelöst in DMSO
- Aufzeichnung der zeitabhängigen Fluoreszenzzunahme über 2 - 10 min.

| **Hemmung von humanen MMPs durch die erfindungsgemäßen Inhibitoren der allgemeinen Formel I** | | | | | |
|---|---|---|---|---|---|
| **Verbindung Nach Bsp.** | **MMP-2** | **MMP-3** | **MMP-8** | **MMP-9** | **MT1-MMP** |
| 1 | 70% Hemmung bei 10 µM | 70% Hemmung bei 10 µM | 70% Hemmung bei 10 µM | 70% Hemmung bei 10 µM | 40% Hemmung bei 10 µM |
| 2 | | | Kᵢ = 60µM | 10% Hemmung bei 10 µM | |
| 3 | 90% Hemmung bei 10 µM | 97% Hemmung bei 10 µM | 87% Hemmung bei 10 µM | 83% Hemmung bei 10 µM | 73% Hemmung bei 10 µM |
| 4 | 50% Hemmung bei 10 µM | 15% Hemmung bei 10 µM | 60% Hemmung bei 10 µM | 50% Hemmung bei 10 µM | 60% Hemmung bei 10 µM |
| 5 | 50% Hemmung bei 10 µM | 90% Hemmung bei 10 µM | 70% Hemmung bei 10 µM | 70% Hemmung bei 10 µM | 80% Hemmung bei 10 µM |
| 6 | | | 43% Hemmung bei 10 µM | 55% Hemmung bei 10 µM | |
| 7 | 20% Hemmung bei 1 µM | Kᵢ = 1,0µM | 20% Hemmung bei 1 µM | 20% Hemmung bei 1 µM | keine Hemmung |
| 8 | 50% Hemmung bei 5µM | 30% Hemmung bei 5 µM | 30% Hemmung bei 5 µM | 30% Hemmung bei 5 µM | 30% Hemmung bei 5 µM |
| 9 | 50% Hemmung bei 3 µM | Kᵢ = 0,25 µM | 50% Hemmung bei 2,5 µM | 50% Hemmung bei 2,5 µM | 50% Hemmung bei 3 µM |
| 10 | 52% Hemmung bei 10 µM | 55% Hemmung bei 10 µM | 39% Hemmung bei 10 µM | 68% Hemmung bei 10 µM | 42% Hemmung bei 10 µM |
| 11 | 15% Hemmung bei 1 µM | Keine Hemmung | 30% Hemmung bei 1 µM | 15% Hemmung bei 1 µM | Keine Hemmung |

## Patentansprüche

1. Substituierte kondensierte Pyrimidin-2,4(1 H,3H)-dione der allgemeinen Formel (I) 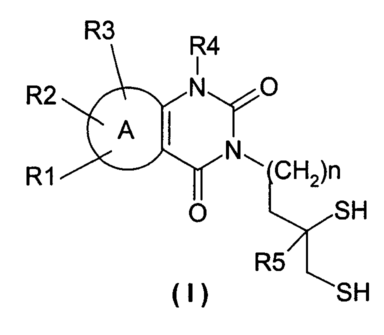 wobei in der Formel I die Indices bedeuten:
n = die Zahlen 0, 1 oder 2,
A = ein anellierter Benzoring oder ein anellierter fünf- bis siebengliedriger Cycloalkylring oder ein anellierter fünf- bis siebengliedriger Cycloheteroalkylring mit einem Sauerstoff- oder Schwefelatom oder ein über zwei Kohlenstoffatome gebundener Heteroaromat mit einem, zwei oder drei Stickstoff-, Sauerstoff- und/oder Schwefelatomen als Heteroatomen,
R1, R2, R3 = unabhängig voneinander Wasserstoff, Halogen, C1-C6 Alkyl, C1-C3-Alkylthio-, C6-C12 Aryl-, Nitro-, Carbamoyl-, C1-C4 Alkoxy, -CN, CF₃, NH₂ -, Carboxy-, C1-C4 Alkoxycarbonyl, C1-C4 N-alkyl-carbamoyl oder C1-C5 Alkenyl
R4 = Wasserstoff, C1-C8 Alkyl, C2-C5 Alkenyl, C2-C8 Alkinyl, C3-C10 Cycloalkyl-, C3-C10 Cycloalkenyl-, C3-C10 Cycloalkinyl-, [-(CH₂)₁₋₅-Z-(C1-C10 Alkyl) mit Z= O, S, C=O, S=O, SO₂], C6-C12 Aryl, C7-C12 Aralkyl,
R5 = Wasserstoff, Methyl

2. Substituierte kondensierte Pyrimidin-2,4(1H,3H)-dione nach Anspruch 1, wobei die für R1 bis R4 definierten Alkyl-, Alkylthio-, Alkoxy-, Alkoxycarbonyl-, N-alkyl-carbamoyl-, Alkenyl-, Alkinyl-, Cycloalkyl-, Cycloalkenyl- und Cycloalkinylgruppen sowie die Z-Gruppe mono oder disubstituiert sind durch Reste aus der Gruppe: Carbamoyl-, Hydroxy, C1-C4 Alkoxy, -CN, SO₃H, CF₃, NH₂, Carboxy-, C1-C4 Alkoxycarbonyl, C1-C4 N-alkyl-carbamoyl, C1-C4 N-dialkyl-carbamoyl, Phenyl oder Piperazinylcarboxy oder einen Heterocyclus, wobei die vorstehend genannten Alkylgruppen substituiert sein können durch ein oder zwei Hydroxygruppen, SO₃H , NH₂, C1-C4 alkylamino, C1-C4 dialkylamino, oder einen Heterocyclus.

3. Pyrimidin-2,4(1H,3H)-dion nach Anspruch 1 oder 2 in Form eines 3-Dimercaptoalkylpyrimidin-2,4(1 H,3H)-dions der allgemeinen Formel I (a) 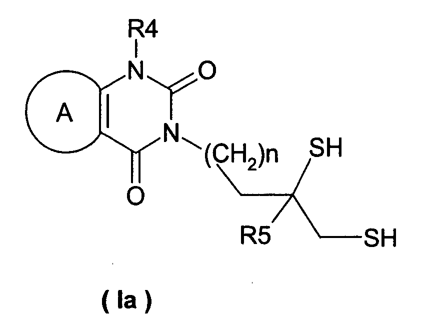
n = die Zahlen 0 oder 1
R4 = Wasserstoff, C1-C8 Alkyl, C2-C5 Alkenyl, C2-C5 Alkinyl, [-(CH₂)₁₋₃-Z-(C1-C5 Alkyl) mit Z= O, S, C=O, S=O, SO₂], C7-C12 Aralkyl, wobei die Alkyl-, Alkenyl-, Alkinylgruppen sowie die Z-Gruppe vorzugsweise mono oder disubstituiert sein können durch Reste aus der Gruppe: Carbamoyl-, Hydroxy, C1-C2 Alkoxy, -CN, SO₃H, CF₃, , Carboxy-, C1-C4 Alkoxycarbonyl, C1-C4 N-alkyl-carbamoyl, C1-C4 N-dialkyl-carbamoyl oder Piperazinylcarboxy und vorstehend genannte Alkylgruppen vorzugsweise substituiert sein können durch ein oder zwei Hydroxygruppen, SO₃H , NH₂, C1-C4 alkylamino, C1-C4 dialkylamino, oder einen Heterocyclus.
R5 = ein Wasserstoffatom oder eine Methylgruppe
A = ein anellierter Benzoring unsubstituiert oder monosubstituiert, disubstituiert
oder trisubstituiert mit Resten, die gleich oder verschiedenartig sein können aus der Gruppe: Halogen, C1 bis C3- Alkyl, C1 bis C3 Alkoxy, Trifluormethyl, Nitro-, Carbamoyl-, -NH₂ , Carboxy-, C1-C4 Alkoxycarbonyl, C1-C4 N-alkyl-carbamoyl, Arylalkylcarbamoyl, Arylalkyloxycarbonyl, Het-alkylcarbamoyl und Het-alkyloxycarbonyl mit Aryl= Phenyl, Tolyl, Pyridyl, Pyrazinyl, Pyrimidinyl, Pyrrolyl, Pyrazolyl, Imidazolyl sowie Het= Morpholinyl, Piperazinyl, Piperidinyl, Pyrrolidinyl, Imidazolidinyl
oder ein anellierter fünf- bis siebengliedriger Cycloalkylring oder ein anellierter fünf- bis siebengliedriger Cycloheteroalkylring mit einem Sauerstoff- oder Schwefelatom
oder ein über zwei Kohlenstoffatome gebundener Heteroaromat, dieser sechsgliedrig mit einem, zwei oder drei Stickstoffatomen als Heteroatomen oder
fünfgliedrig mit einem Stickstoffatom oder fünfgliedrig mit einem Sauerstoff - oder Schwefelatom oder fünfgliedrig mit einem Stickstoffatom sowie einem Sauerstoff- oder einem Schwefelatom oder
fünfgliedrig mit zwei Stickstoffatomen als Heteroatomen oder
fünfgliedrig mit zwei Stickstoffatomen und einem Schwefelatom oder
fünfgliedrig mit drei Stickstoffatomen als Heteroatomen,
wobei die vorstehend für A definierten Heteroaromaten durch Reste R6 mono-, di- oder trisubstituiert und die Substituenten gleich oder verschieden sein können
R6 = Halogen, C1 bis C6- Alkyl, C1 bis C3-Alkylthio, Cyano, Trifluormethyl, Benzyl, Phenyl, wobei die beiden letztgenannten aromatischen Reste wie folgt substituiert sein können:
C1 bis C3-Alkyl, Halogen, OR7, COR7, SO2R7, OCOR8, COOR8, CONR8R9, SO2NR8R9, NR8R9 mit
R7 = H, C1 bis C4-Alkyl, C3 bis C6-Cycloalkyl, Phenyl,
R8/R9 = unabhängig voneinander H, Benzyl, letzteres unsubstituiert oder durch Methoxy mono-, di- oder trisubstituiert, C1 bis C4-Alkyl oder R8/R9 = als geradkettige Alkylgruppe gemeinsam einen Ring bildend mit einem Stickstoffatom einen fünfgliedrigen Ring, der ggf. ein weiteres Heteroatom N, O, S enthalten kann, oder gemeinsam einen sechsgliedrigen Heterocyclus bildend mit einem Stickstoffatom sowie ggf. einem weiteren Stickstoffatom oder einem Sauerstoffatom oder einem Schwefelatom, wobei Fünf- und/oder Sechsring im Kohlenstoffgerüst mit C1 bis C4 Alkyl oder mit Benzyl substituiert sein können
R6 = für den Fall, daß A Thieno ist, außerdem ein anellierter C4- bis C7- Cycloalkylring, der ein Sauerstoff, ein Schwefel- oder ein Stickstoffatom enthalten kann, wobei das Stickstoffatom unsubstituiert oder substituiert ist mit C1 bis C4 Alkyl- , Phenyl oder Benzyl und die beiden letztgenannten Aromaten mit C1 bis C3 Alkyl, C1 bis C3 Alkoxy oder Halogen substituiert sein können, ferner kann für den Fall A= Thieno R6 außerdem sein: Carboxy, eine mit einem Heterocyclus substituierte Alkylgruppe C1-C5, ein Heterocyclus sowie eine C1-C5 N-Alkylcarbamoylgruppe, die mit Amino, Sulfo, Hydroxy, Dihydroxy oder einem Heterocyclus substituiert sein kann.

4. 3-Dimercaptoalkylpyrimidin-2,4(1H,3H)-dion nach Anspruch 1, 2 oder 3, **dadurch gekennzeichnet, daß** es sich um folgende Verbindungen handelt
(R,S)-3-(2,3-Dimercaptopropyl)chinazolin-2,4-(1H,3H)dion
(R,S)-3-(2,3-Dimercapto-2-methylpropyl)chinazolin-2,4-(1H,3H)dion
(R,S)-7-Chlor-3-(2,3-dimercaptopropyl)chinazolin-2,4-(1H,3H)dion
(R,S)-6-Chlor-3-(2,3-dimercaptopropyl)chinazolin-2,4-(1H,3H)dion
(R,S)-3-(2,3-Dimercaptopropyl)-5-methyl-chinazolin-2,4-(1H,3H)dion
(R,S)-3-(2,3-Dimercaptopropyl)-8-methyl-chinazolin-2,4-(1H,3H)dion
(R,S)-3-(2,3-Dimercaptopropyl)-6,7-dimethoxy-chinazolin-2,4-(1H,3H)dion
(R,S)-N-Benzyl-3-(2,3-dimercaptopropyl)-2,4-dioxo-1,2,3,4-tetrahydrochinazolin-7-carboxamide
(R,S)-3-(2,3-dimercaptopropyl)pyrido[3,2-d]pyrimidin-2,4(1H,3H)-dion
(R,S)-3-(2,3-dimercapto-2-methyl-propyl)pyrido[ 3;2-d]pyrimidin-2,4(1H,3H)-dion
(R,S)-3-(2,3-dimercaptopropyl)pyrido[2,3-d]pyrimidin-2,4(1H,3H)-dion
(R,S)-3-(2,3-dimercapto-2-methyl-propyl)pyrido[2,3-d]pyrimidin-2,4(1H,3H)-dion
(R,S)-3-(2,3-dimercaptopropyl)pteridin-2,4(1H,3H)-dion
(R,S)-3-(2,3-dimercapto-2-methyl-propyl)pteridin-2,4(1H,3H)-dion
(R,S)-5-(2,3-dimercaptopropyl)-2H-pyrazolo[3,4-d]pyrimidin-4,6(5H,7H)-dion
(R,S)-5-(2,3-dimercapto-2-methyl-propyl)-2H-pyrazolo[3,4-d]pyrimidin-4,6(5H,7H)-dion
(R,S)-5-(2,3-dimercaptopropyl)-1H-pyrazolo[3,4-d]pyrimidin-4,6(5H,7H)-dion
(R,S)-5-(2,3-dimercapto-2-methyl-propyl)-1H-pyrazolo[3,4-d]pyrimidin-4,6(5H,7H)-dion
(R,S)-3-(2,3-dimercaptopropyl)-5-methyl-1H-pyrrolo[3,2-d]pyrimidin-2,4(3H,5H)-dion
(R,S)-3-(2,3-dimercapto-2-methylpropyl)-5-methyl-1H-pyrrolo[3,2-d]pyrimidin-2,4(3H,5H)-dion
(R,S)-3-(2,3-dimercaptopropyl)-1-methylchinazolin-2,4(1H,3H)-dion
(R,S)-3-(2,3-dimercapto-2-methylpropyl)-1-methylchinazolin-2,4(1H,3H)-dion
(R,S)-3-(2,3-Dimercaptopropyl)thieno[3,2-d]pyrimidin-2,4(1H,3H)-dion
(R,S)-3-(2,3-Dimercaptopropyl)-5,6,7,8-tetrahydro[1]benzothieno[2,3-d]pyrimidin-2,4(1 H,3H)-dion
(R,S)-3-(2,3-Dimercaptopropyl)-6-phenylthieno[2,3-d]pyrimidin-2,4(1H,3H)-dion

5. Verfahren zur Herstellung von 3-Dimercaptoalkylpyrimidin-2,4(1H,3H)-dionen nach einem der Ansprüche 1 bis 4, **dadurch gekennzeichnet, daß**
Monomercaptane der allgemeinen Formel II 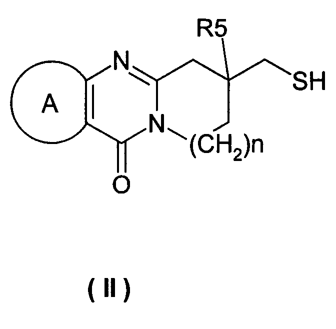
n = die Zahlen 0 oder 1,
A = ein anellierter Benzolring oder ein anellierter fünf- bis siebengliedriger Cycloalkylring oder ein anellierter fünf- bis siebengliedriger Cycloheteroalkylring mit mindestens einem Sauerstoff- oder Schwefelatom oder ein über zwei Kohlenstoffatome gebundener fünf- bis siebengliedriger Heteroaromat mit einem, zwei oder drei Stickstoff-, Sauerstoff- und/oder Schwefelatomen als Heteroatomen, wobei A unsubstituiert, mono-, di- oder trisubstituiert ist mit Halogen, C1-C6 Alkyl, C1-C3- Alkylthio-, C6-C12-Aryl-, Nitro-, Carbamoyl-, C1-C4 Alkoxy, -CN, CF₃, NH₂ -, Carboxy-, C1-C4 Alkoxycarbonyl, C1-C4 N-alkyl-carbamoyl oder C1-C5 Alkenyl
R5 = Wasserstoff, Methyl
einer sauren Hydrolyse derart unterworfen werden, daß eine Monomercaptanverbindung der allgemeinen Formel II in eine Säuremischung aus Schwefelsäure, Eisessig und Wasser eingetragen und 8 bis 10 Stunden erhitzt wird, nach dem Abkühlen Wasser zugefügt und der Niederschlag separiert wird.

6. Verfahren zur Herstellung von 3-Dimercaptoalkylpyrimidin-2,4(1H,3H)-dionen nach einem der Ansprüche 1 bis 3, **dadurch gekennzeichnet, daß** Monomercaptane der allgemeinen Formel II 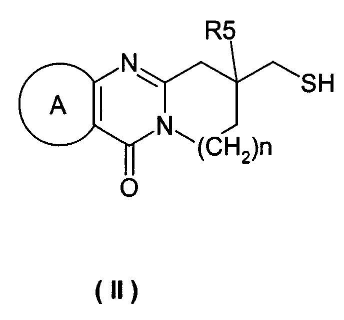
n = die Zahlen 0 oder 1,
A = ein anellierter Benzolring oder ein anellierter fünf- bis siebengliedriger Cycloalkylring oder ein anellierter fünf- bis siebengliedriger Cycloheteroalkylring mit mindestens einem Sauerstoff- oder Schwefelatom oder ein über zwei Kohlenstoffatome gebundener fünf- bis siebengliedriger Heteroaromat mit einem, zwei oder drei Stickstoff-, Sauerstoff- und/oder Schwefelatomen als Heteroatomen, wobei A unsubstituiert, mono-, di- oder trisubstituiert ist mit Halogen, C1-C6 Alkyl, C1-C3- Alkylthio-, C6-C12-Aryl-, Nitro-, Carbamoyl-, C1-C4 Alkoxy, -CN, CF₃, NH₂ -, Carboxy-, C1-C4 Alkoxycarbonyl, C1-C4 N-alkyl-carbamoyl oder C1-C5 Alkenyl
R5 = Wasserstoff, Methyl
einer alkalischen Hydrolyse derart unterzogen werden, daß eine Monomercaptanverbindung der allgemeinen Formel II
in einer inerten Atmosphäre in eine Mischung aus 3N NaOH/Ethanol (10:1) und 100 mg Zinkstaub eingetragen wird, unter weiterem Einleiten von Stickstoff bis zum vollständigen Umsatz bei 80 bis 85 Grad C gerührt wird und der erkaltete Reaktionsansatz unter Kühlung und in inerter Atmosphäre in 10 %-ige Salzsäure filtriert und der Niederschlag separiert wird.

7. Verfahren nach Anspruch 5 oder 6, wobei die jeweiligen Verfahrenprodukte nachträglich an der N1-Position mit einem Rest R4 = C1-C8 Alkyl, C2-C5 Alkenyl, C2-C8 Alkinyl, C3-C10 Cycloalkyl-, C3-C10 Cycloalkenyl-, C3-C10 Cycloalkinyl-, [-(CH₂)₁₋₅ - Z-(C1-C10 Alkyl) mit Z= O, S, C=O, S=O, SO₂], C6-C12 Aryl, C7-C12 Aralkyl subsituiert werden.

8. Verwendung von substituierten kondensierten Pyrimidin-2,4(1H,3H)-dionen gemäß einem der Ansprüche 1 bis 4 zur Herstellung eines Arzneimittels zur Behandlung von Krebs, Rheuma, Entzündungen und allergischen Reaktionen.

9. Verwendung nach Anspruch 8, wobei es sich um die 3-Dimercaptoalkylpyrimidin-2,4(1 H,3H)-dione gemäß Anspruch 3 oder 4 handelt.
